(19) Europäisches Patentamt
European Patent Office
Office européen des brevets

(11) Veröffentlichungsnummer : **0 038 007**
**B1**

(12) # EUROPÄISCHE PATENTSCHRIFT

(45) Veröffentlichungstag der Patentschrift : **15.12.82**

(51) Int. Cl.³ : **C 07 D263/44**// C07C125/065

(21) Anmeldenummer : **81102596.4**

(22) Anmeldetag : **07.04.81**

(54) **Verfahren zur Herstellung von N-(3,5-Dichlorphenyl)-oxazolidin-2,4-dionen.**

(30) Priorität : **12.04.80 DE 3014119**

(43) Veröffentlichungstag der Anmeldung : **21.10.81 (Patentblatt 81/42)**

(45) Bekanntmachung des Hinweises auf die Patenterteilung : **15.12.82 Patentblatt 82/50**

(84) Benannte Vertragsstaaten : **AT BE CH DE FR GB IT LI NL SE**

(56) Entgegenhaltungen : **DE C 729 850**

(73) Patentinhaber : **BASF Aktiengesellschaft**
**Carl-Bosch-Strasse 38**
**D-6700 Ludwigshafen (DE)**

(72) Erfinder : **Zanker, Fritz, Dr.**
**Denkstrasse 27**
**D-6520 Worms (DE)**
Erfinder : **Scholz, Herbert, Dr,**
**Im Finkenschlag**
**D-6730 Neustadt (DE)**
Erfinder : **Keller, Peter, Dr.**
**Brunnengasse 24**
**D-6940 Weinheim (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von N-(3,5-Dichlorphenyl)-oxazolidin-2,4-dionen

Die Erfindung betrifft ein neues Verfahren zur Herstellung der in DE-OS 22 07 576 und DE-OS 23 24 591 beschriebenen N-(3,5-Dichlorphenyl)-oxazolidin-2,4-dione aus 3,5-Dichlorphenylcarbamoyl-oxy-essigsäureestern in Alkoholen in Gegenwart von basischen Katalysatoren.

Oxazolidin-2,4-dione können nach verschiedenen bekannten Verfahren hergestellt werden. Das wirtschaftlich interessanteste Verfahren ist die basenkatalysierte, intramolekulare Cyclisierung von Carbamoyloxy-essigsäureestern unter Abspaltung von Alkohol in einem inerten Lösungsmittel wie Benzol, Toluol, Dichlorbenzol, Nitrobenzol etc. Die Umsetzung sei für die Herstellung von N-Phenyl-5-methyl-oxazolidin-2,4-dion aus Phenylcarbamoyloxy-methylessigsäureäthylester formelhaft wiedergegeben

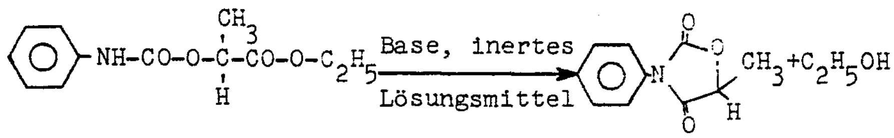


Das Arbeiten in Gegenwart eines inerten Lösungsmittels bringt im Vergleich zum Arbeiten in Abwesenheit eines Lösungsmittels Vorteile mit sich, wie eine höhere Ausbeute und damit umso größere Kosteneinsparungen, je teuerer die Einsatzstoffe sind, oder eine betriebssicherere Arbeitsweise durch Vermeidung von Apparate- und Rohrleitungsverstopfungen durch unerwünschtes Festwerden der Reaktionsteilnehmer oder durch Abfuhr eventuell auftretender Reaktionswärme durch Siedekühlung durch verdampfendes und an einem Rückflußkühler kondensierendes inertes Lösungsmittel. Letztlich ist die Verwendung eines Lösungsmittels bei der Aufarbeitung der Reaktionsmischung und der Reinigung der Oxazolidin-2,4-dione umso wichtiger, je größer die Anforderung an deren Reinheit ist. Diese ist im Falle der Verwendung der N-(3,5-Dichlorphenyl)-oxazolidin-2,4-dione als Pflanzenschutzmittelwirkstoffe besonders hoch, so daß sich die Verwendung eines die Aufarbeitung der Reaktionsmischung und die Reinigung der N-3,5-Dichlorphenyl-oxazolidin-2,4-dione erleichternden inerten Lösungsmittels für die Durchführung der chemischen Umsetzung geradezu anbiedet.

Die üblichen inerten organischen Lösungsmittel, in denen die Oxazolidin-2,4-dione hergestellt werden, erleichtern die Aufarbeitung des Reaktionsansatzes und die Reinigung der entstandenen Oxazolidin-2,4-dione nicht ; im Gegenteil, durch die gewöhnlich gute Löslichkeit der letzteren wirken sie geradezu hemmend, da sie zwecks Aufarbeitung der Reaktionsmischung und Reinigung der Oxazolidin-2,4-dione zusammen mit dem bei der Cyclisierung entstandenen Alkohol erst destillativ aus der Reaktionsmischung entfernt werden müssen.

Die Verwendung von inerten organischen Lösungsmitteln wie Toluol, Xylol, Benzol, Chlorbenzol, Dichlorbenzol, Äthylenchlorid, Cyclohexan, Octan, Hexan, Chloroform, Essigsäureäthylester etc ist aber auch deshalb nicht zu empfehlen, weil sie mit dem bei der Herstellung der Oxazolidin-2,4-dione entstehenden Alkohol azeotrope Gemische bilden (vgl. Taschenbuch für Chemiker und Physiker, herausgegeben von Dr.-Ing. Jean D'Ans und Dr. phil. Ellen Lax (1943), Seiten 903 und 904, und Azeotropic data, number six of the Advances in Chemistry Series (Edited by the staff of Industrial and Engineering Chemistry, Published June 1952 by AMERICAN CHEMICAL SOCIETY), so daß das destillativ zurückgewonnene und gereinigte Lösungsmittel oder der destillativ zurückgewonnene und gereinigte Alkohol infolge der Verunreinigung mit dem anderen Partner für die Wiederverwendung für eine erneute Umsetzung ungeeignet ist. Das gilt z.B. für das mit dem Alkohol verunreinigte inerte Lösungsmittel bei dem üblichen Verfahren der Herstellung der Oxazolidin-2,4-dione aus Isocyanaten und Glykolsäureestern zu den Carbamoyloxy-essigsäureestern und der anschließenden basenkatalysierten Cyclisierung ohne Wechsel des Lösungsmittels, da der Alkohol im inerten Lösungsmittel bei dessen Wiederverwendung bevorzugt mit den Isocyanaten reagiert oder z.B. für den mit inertem Lösungsmittel verunreinigten Alkohol bei der Herstellung der Glykolsäureester. Neben der Menge des azeotropen Gemisches kann die Menge des destillativ rein zurückgewonnenen, wiedereinsetzbaren Lösungsmittels oder Alkohols so gering sein, daß die Aufarbeitung überhaupt nicht mehr sinnvoll ist.

Es wurde nun gefunden, daß man N-(3,5-Dichlorphenyl)-oxazolidin-2,4-dione der Formel

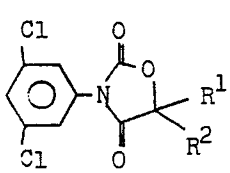

in der $R^1$ Halogenalkanyl, Alkenyl, Chlormethyl, CN, die CO-O-Alkylgruppe mit 2 bis 5 Kohlenstoffatomen, Alkoxyalkyl oder Alkylthioalkyl mit je 2 bis 4 Kohlenstoffatomen und $R^2$ Halogenalkyl, Alkenyl,

Wasserstoff oder einen Alkylrest mit 1 bis 4 Kohlenstoffatomen bedeuten, durch Erhitzen von 3,5-Dichlorphenylcarbamoyloxyessigsäureestern der Formel

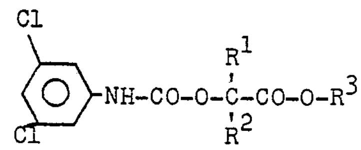

in der $R^1$ und $R^2$ die oben genannten Bedeutungen haben und $R^3$ einen Alkylrest mit 1 bis 10 Kohlenstoffatomen oder einen Cyclohexylrest bedeutet, in Gegenwart eines basischen Katalysators bei Temperaturen zwischen 20 und 200 °C und Abspaltung von Alkohol der Formel

$R^3$—OH

in der $R^3$ die oben genannte Bedeutung, in einfacher Weise erhält, wenn man das Erhitzen in Gegenwart von einem Alkohol der Formel

$R^4$—OH

durchführt, in der $R^4$ einen Alkylrest mit 1 bis 10 Kohlenstoffatomen, Hydroxy-äthyl oder einen Cyclohexylrest bedeutet und gleich oder verschieden von $R^3$ ist, und die in fester Form aus der Reaktionsmischung ausfallenden Oxazolidin-2,4-dione nach bekannten Verfahren bei Temperaturen zwischen − 30°.und + 90 °C von der Reaktionsmischung abtrennt.

Es war nicht zu erwarten, daß die Umsetzung der Carbamoyloxy-essigsäureester zu den Oxazolidin-2,4-dionen in den Alkoholen $R^4$ OH mit besserer Ausbeute als in den inerten Lösungsmitteln verläuft, in denen die bekannte bevorzugte Herstellung der Oxazolidin-2,4-dione durch Addition von Glykolsäureestern an 3,5-Dichlorphenylisocyanat und die basenkatalysierte Cyclisierung der entstandenen 3,5-Dichlorphenylcarbamoyloxy-essigsäureester durchgeführt wird. Lambling (M.E. Lambling, Bull. Soc. Chim. (3) *27*, 441-451 (1902)) hat nämlich gezeigt, daß zwischen N-Phenyloxazolidin-2,4-dion, Wasser und Phenylcarbamoyloxy-essigsäure das selbst bei Raumtemperatur sich einstellende Gleichgewicht

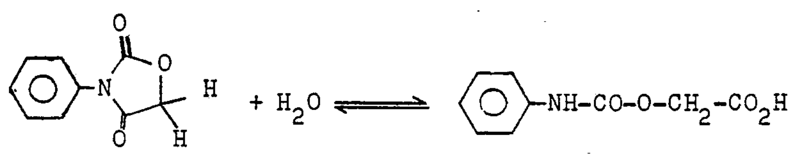

besteht, so daß auch ein entsprechendes Gleichgewicht zwischen den entsprechenden Estern und Alkoholen, zumal in Gegenwart eines basischen Katalysators gemäß den Formeln

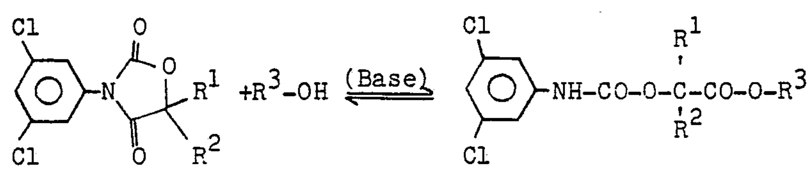


erwartet werden mußte. Die Umsetzung der Carbamoyloxy-essigsäureester zu den Oxazolidin-2,4-dionen in einem Alkohol $R^4$—OH sollte zu einem analogen Gleichgewicht mit $R^4$ anstelle von $R^3$ führen und damit die Ausbeute an den gewünschten Oxazolidin-2,4-dionen stark mindern. Überraschend ist das aber nicht so.

Die Verwendung eines Alkohols als Lösungsmittel macht im Gegensatz zur bekannten Verwendung inerter Lösungsmittel, die die Oxazolidin-2,4-dione der Formel I gut lösen, deren Aufarbeitung und Reinigung besonders leicht, da sie in einfacher Weise, z.B. durch bloßes Absaugen aus der Reaktionsmischung, in sehr größer Reinheit direkt gewonnen werden können.

Zusätzlich zu den erhöhten Ausbeuten an den Dionen und ihrer einfachen Abtrennung durch direkte Absaugung aus dem Reaktionsgemisch kommt der Vorteil der gegenüber den bekannten Verfahren einfacheren und weit kostengünstigeren Lösungsmittelrückgewinnung nach dem erfindungsgemäßen Verfahren : Der Lösungsmittelalkohol $R^4$—OH und der bei der Reaktion entstandene Alkohol $R^3$—OH bilden kein azeotropes Gemisch und können durch Destillation leicht voneinander getrennt und damit rein zurückgewonnen werden, bzw. $R^4$—OH und $R^3$—OH brauchen für den Fall, daß $R^4$ und $R^3$ identisch

sind, destillativ überhaupt nicht getrennt zu werden. Die Alkohole können danach ohne Schwierigkeiten erneut für die gleiche Umsetzung verwendet werden.

Bevorzugte Substituenten $R^1$, $R^2$, $R^3$ und $R^4$ sind : $R^1$ Vinyl und Methoxymethyl, $R^2$ Methyl, $R^3$ und $R^4$ Alkyl mit 1 bis 4 Kohlenstoffatomen, wie Äthyl, n-Propyl, i-Propyl, N-Butyl, sek.-Butyl, tert.-Butyl, insbesondere Methyl und i-Butyl.

Die Umsetzung wird vorteilhaft in 10 bis 5 000 Gewichtsprozenten Lösungsmittelalkohol der Formel $R^4$—OH, insbesondere in 20 bis zu 500 Gewichtsprozenten, bezogen auf eingesetzten Carbamoyloxyessigsäureester, durchgeführt.

Als basische Katalysatoren kommen in Betracht : Alkoholate wie Natriummethylat, Alkalioxide wie Natriumoxid, Erdalkalioxide wie Calciumoxid, Alkalicarbonate wie Kaliumcarbonat, Alkalihydrogencarbonate wie Natriumbicarbonat, Erdalkalicarbonate wie Calciumcarbonat, Fluoride wie Kaliumfluorid, Amine wie Ammoniak, Methylamin, Äthylamin, Dimethylamin, insbesondere tertiäre Amine wie Triäthylamin, Tripopylamin, Pyridin, N,N-Dimethylanilin. Die eingesetzte Menge des Katalysators kann bis zu 50 Gewichtsprozent, bezogen auf eingesetzten Alkohol $R^4$—OH betragen, liegt aber im Regelfall bei 1 bis 10 Gewichtsprozent.

Die Umsetzung der Carbamoyloxi-essigsäureester zu den Oxazolidin-2,4-dionen erfolgt gewöhnlich bereits bei Raumtemperatur. Vorteilhaft sind jedoch Temperaturen zwischen 35 und 200 °C, insbesondere solche zwischen 45 und 150 °C.

Die Trennung der in fester Form aus der Reaktionslösung ausfallenden Oxazolidin-2,4-dione nach beendeter Umsetzung, z.B. durch bloßes Absaugen, erfolgt bei Temperaturen zwischen − 30 und + 90 °C, vorzugsweise zwischen − 10 und + 50 °C.

Die folgenden Beispiele (außer Beispiel A) erläutern die Durchführung des erfindungsgemäßen Verfahrens. Darin bedeuten die aufgeführten Teile Gewichtsteile. Sie verhalten sich zu den Volumenteilen wie Kilogram zu Liter.

## Beispiel A (bekanntes Verfahren)

Zu einer homogenen Mischung aus 2 500 Teilen 3,5-Dichlor-phenylisocyanat und 2 480 Teilen Toluol läßt man bei einer Temperatur von 120-128 °C 2 287 Teile Vinylmilchsäure-i-butyl-ester unter Rühren innerhalb von 3-4 Stunden zulaufen und rührt bei dieser Temperatur 1 Stunde nach. Die Bildung des 3,5-Dichlorphenylcarbamoyloxy-methyl-vinyl-essigsäure-i-butylesters ist dann beendet. Man kühlt auf ca. 110 °C ab, gibt 107 Teile Tri-n-propylamin zu und rührt bei ca. 110 °C 1 Stunde nach. Man destilliert nun über einen Kühler 3 360 Teile eines Gemisches aus 2 405 Teilen Toluol und 955 Teilen des nach der Zugabe von Tri-n-propylamin entstandenen i-Butylalkohols im Vakuum, zuletzt bei ca. 90 °C im Ansatzbehälter und 50 mbar Druck ab.

Der Rückstand wird in 2 740 Teilen Methanol unter Kühlung eingerührt, die enstandene Suspension bei 15-20 °C abgesaugt und das Festprodukt mit 400 Teilen Methanol gewaschen.

Mutterlauge und Waschmethanol werden gesammelt und das Methanol abdestilliert. Man erhält 2 380 Teile wieder einsetzbares Methanol, entsprechend einem Verlust von ca. 17 % Methanol pro Ansatz.

Das Festprodukt wird mit Wasser methanolfrei gewaschen und getrocknet. Ausbeute 3 384 Teile 99 %iges N-(3,5-Dichlorphenyl)-5-methyl-5-vinyl-oxazolidin-2,4-dion, entsprechend 88 % d. Th. Schmp. 106-108 °C.

Das azeotrope Gemisch aus Toluol und i-Butanol besteht bei Normaldruck zu 55,5 % aus Toluol und hat einen niederen Siedepunkt (101,2 °C) als die Einzelkomponenten Toluol (Sdp. 110,6 °C) und i-Butanol (108,3 °C). Würde man daher die obigen 3 360 Teile Destillat fraktionieren, dann erhielte man ein nicht wieder einsetzbares azeotropes Gemisch, bestehend aus 955 Teilen i-Butanol und 1 191 Teilen Toluol. Lediglich 2 405-1 191 = 1 214 Teile wieder einsetzbares Toluol könnten theoretisch destillativ zurückgewonnen werden. Der Gesamtverlust an Rein-i-Butanol ist vollständig.

## Beispiel 1

Zu einer homogenen Mischung aus 2 500 Teilen 3,5-Dichlorphenylisocyanat und 2 480 Teilen Toluol läßt man bei einer Temperatur von 120-128 °C 2 287 Teile Vinylmilchsäure-i-butylester unter Rühren innerhalb von 3-4 Stunden zulaufen und rührt bei dieser Temperatur 1 Stunde nach. Man destilliert nun über einen Kühler 2 360 Teile Toluol zuletzt bei 90 °C im Ansatzbehälter und 50 mbar Druck ab.

Der eingeengte flüssige Rückstand, bestehend aus dem 3,5-Dichlorphenylcarbamoyloxy-methyl-vinyl-essigsäure-i-butylester, wird in eine Lösung von 107 Teilen Tri-n-propylamin in 2 470 Teilen Methanol, eventuell unter Kühlung, eingerührt. Man erhitzt 6 Stunden am Rückfluß, kühlt die entstandene Suspension auf 15-20 °C ab, saugt ab und wäscht das Festprodukt mit 400 Teilen Methanol. Mutterlauge und Waschmethanol werden gesammelt. Anschließend wird das Festprodukt mit Wasser methanol- und i-butanolfrei gewaschen und getrocknet. Ausbeute 3 496 Teile 99 %iges N-(3,5-Dichlorphenyl)-5-methyl-5-vinyl-oxazolidin-2,4-dion, entsprechend 91 % d. Th., Schmp. 106-108 °C.

Obige 2 360 Teile zurückgewonnenes Toluol sind ohne Reinigung für den nächsten Ansatz verwendbar. Verlust an Toluol pro Ansatz : ca. 5 %.

Methanol und i-Butanol bilden kein azeotropes Gemisch. Die Fraktionierung obiger Mutterlauge und

des obigen Waschmethanols ergibt 844 Teile für die Synthese von Vinylmilchsäure-i-butylester einsetzbares i-Butanol, entsprechend einem Verlust von ca. 14 % bezogen auf die theoretisch zurückgewinnbare i-Butanolmenge, und 2 490 Teile wieder einsetzbares Methanol, entsprechend einem Verlust von ca. 13 % Methanol pro Ansatz.

Beispiel 2

321 Teile 3,5-Dichlorphenylcarbamoyloxi-methyl-methoximethylessigsäure-i-butylester (durch Addition von 1-Methyl-1-methoxy-methyl-glykolsäure-i-butylester an 3,5-Dichlorphenylisocyanat erhalten) werden in einer Lösung von 7 Teilen Tri-n-propylamin in 163 Teilen Methanol 8 Stunden am Rückfluß gekocht. Nach dem Abkühlen auf 15-20 °C saugt man auf einer Nutsche ab und wäscht den festen Rückstand mit 30 Teilen Methanol.

Die Mutterlauge und das Waschmethanol werden gesammelt.

Anschließend wird das Festprodukt mit Wasser methanol- und i-butanolfrei gewaschen und getrocknet. Ausbeute 232 g N-(3,5-Dichlorphenyl)-5-methyl-5-methoxymethyl-oxazolidin-2,4-dion, entsprechend 90 % d. Th., Schmp. 111-113 °C.

Aus der Mutterlauge und dem Waschmethanol werden destillativ 86 % des theoretisch zurückgewinnbaren i-Butanols mit für die Synthese von 1-Methyl-1-methoxymethyl-glykolsäure-i-butylester geeigneter Qualität und 85 % wieder einsetzbares Methanol zurückgewonnen.

Beispiel 3

270 Teile 3,5-Dichlorphenylcarbomoyloxy-methyl-vinyl-essigsäure-methylester (durch Addition von Vinylmilchsäuremethylester an 3,5-Dichlorphenylisocyanat erhalten) werden in einer Lösung von 9 Teilen Tri-n-butylamin in 170 Teilen Methanol 8 Stunden am Rückfluß gekocht. Nach dem Abkühlen auf 15-20 °C saugt man auf einer Nutsche ab und wäscht den Rückstand mit 30 Teilen Methanol.

Die Mutterlauge und das Waschmethanol werden gesammelt.

Anschließend wird das Festprodukt mit Wasser methanolfrei gewaschen und getrocknet. Ausbeute 216 g N-(3,5-Dichlorphenyl)-5-methyl-5-vinyl-oxazolidin-2,4-dion, entsprechend 89 % d. Th., Schmp. 106-108 °C.

Durch Destillation der Mutterlauge und des Waschmethanols können 109 Teile in die Synthese von Vinylmilchsäuremethylester und als Lösungsmittel für obige Reaktion einsetzbares Methanol, entsprechend 84 % der theoretisch zu erwartenden Menge, zürückgewonnen werden.

Beispiel 4

Zu 160 Teilen 3,5-Dichlorphenylisocyanat werden bei 100 °C 146 Teilen Vinylmilchsäure-i-butylester gegeben. Das Gemisch wird 3 Stunden bei dieser Temperatur und anschließend 2 Stunden bei 125 °C nachgerührt. Die Umsetzung zum 3,5-Dichlorphenylcarbamoyloxy-methyl-vinyl-essigsäure-i-butylester ist danach beendet.

Man kühlt auf 70 °C, läßt eine Lösung von 7 Teilen Tri-n-propylamin in 163 Teilen Methanol zulaufen, kocht 6 Stunden am Rückfluß und kühlt auf 15-20 °C ab. Man saugt auf einer Nutsche ab und wäscht den Rückstand mit 20 Teilen Methanol.

Die Mutterlauge und das Waschmethanol werden gesammelt.

Anschließend wird das Festprodukt mit Wasser methanol- und i-butanolfrei gewaschen und getrocknet. Ausbeute 221 Teilen 99 %iges N-(3,5-Dichlorphenyl)-5-methyl-5-vinyl-oxazolidin-2,4-dion entsprechend 90 % d. Th., Schmp. 106-108 °C.

Die Regenerierung von Lösungsmittel aus der Umsetzung von Vinylmilchsäure-i-butylester mit 3,5-Dichlorphenylisocyanat entfällt, da hierbei lösungsmittelfrei gearbeitet wurde.

Aus der Mutterlauge und dem Waschmethanol werden destillativ 86 % des theoretisch zurückgewinnbaren i-Butanols mit für die Synthese von Vinylmilchsäure-i-butylester geeigneter Qualität und 85 % wiedereinsetzbares Methanol zurückgewonnen.

## Ansprüche

1. Verfahren zur Herstellung von Oxazolidin-2,4-dionen der Formel

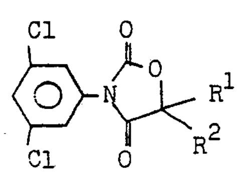

in der $R^1$ Halogenalkenyl, Alkenyl, Chlormethyl, CN, und die CO-O-Alkylgruppe mit 2 bis 5 Kohlenstoffatomen, Alkoxyalkyl oder Alkylthioalkyl mit je 2 bis 4 Kohlenstoffatomen und $R^2$ Halogenalkenyl, Alkenyl, Wasserstoff oder einen Alkylrest mit 1 bis 4 Kohlenstoffatomen bedeuten, durch Erwärmen von 3,5-Dichlorphenylcarbamoyloxy-essigsäureestern der Formel

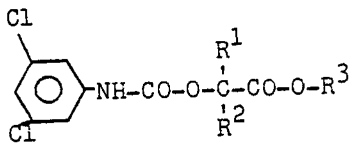

in der $R^1$ und $R^2$ die oben genannten Bedeutungen haben und $R^3$ einen Alkylrest mit 1 bis 10 Kohlenstoffatomen oder einen Cyclohexylrest bedeutet, in Gegenwart eines basischen Katalysators bei Temperaturen zwischen 20 und 200 °C und Abspaltung von Alkohol der Formel

$$R^3—OH$$

in der $R^3$ die oben genannte Bedeutung hat, dadurch gekennzeichnet, daß man das Erwärmen in Gegenwart von einem Alkohol der Formel

$$R^4—OH$$

durchführt, in der $R^4$ einen Alkylrest mit 1 bis 10 Kohlenstoffatomen, Hydroxy-äthyl oder einen Cyclohexylrest bedeutet und gleich oder verschieden von $R^3$ ist, und die in fester Form aus der Reaktionsmischung ausfallenden Oxazolidin-2,4-dione nach bekannten Verfahren bei Temperaturen zwischen − 30 und + 90 °C von der Reaktionsmischung abtrennt.

2. Verfahren nach Anspruch 1, dadurch gekennzeichnet, daß man die Umsetzung mit Verbindungen durchführt, in der $R^1$ Vinyl oder Methoxymethyl, $R^2$ Methyl und $R^3$ Alkyl mit 1 bis 4 Kohlenstoffatomen bedeutet.

3. Verfahren nach Anspruch 1, dadurch gekennzeichnet, daß $R^4$ Alkyl mit 1 bis 4 Kohlenstoffatomen bedeutet.

4. Verfahren nach Anspruch 1, dadurch gekennzeichnet, daß man als Katalysator ein tertiäres Amin verwendet.

**Claims**

1. A process for the preparation of oxazolidine-2,4-diones of the formula

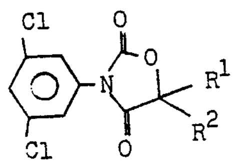

where $R^1$ is haloalkenyl, alkenyl, chloromethyl, CN, CO-O-alkyl of 2 to 5 carbon atoms or alkoxyalkyl or alkylthioalkyl, each of 2 to 4 carbon atoms, and $R^2$ is haloalkenyl, alkenyl, hydrogen or alkyl of 1 to 4 carbon atoms, by heating a 3,5-dichlorophenylcarbamyloxyacetic acid ester of the formula

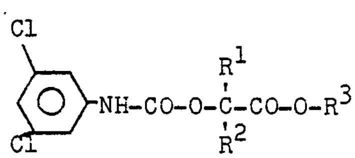

where $R^1$ and $R^2$ have the above meanings and $R^3$ is alkyl of 1 to 10 carbon atoms or cyclohexyl, in the presence of a basic catalyst, at from 20° to 200 °C, with elimination of an alcohol of the formula

$$R^3—OH$$

where $R^3$ has the above meanings, wherein heating is carried out in the presence of an alcohol of the formula

$R^4$—OH

where $R^4$ is alkyl of 1 to 10 carbon atoms, hydroxyethyl or cyclohexyl and is identical with or different from $R^3$, and the oxazolidine-2,4-dione which precipitates as a solid from the reaction mixture is isolated from the latter by conventional methods at from − 30° to + 90 °C.

2. A process as claimed in claim 1, wherein the reaction is carried out with compounds wherein $R^1$ is vinyl or methoxymethyl, $R^2$ is methyl and $R^3$ is alkyl of 1 to 4 carbon atoms.

3. A process as claimed in claim 1, wherein $R^4$ is alkyl of 1 to 4 carbon atoms.

4. A process as claimed in claim 1, wherein the catalyst used is a tertiary amine.

**Revendications**

1. Procédé de préparation d'oxazolidine-2,4-diones de formule

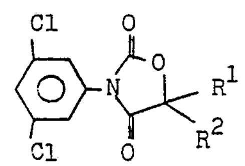

dans laquelle $R^1$ représente halogénalcényle, alcényle, chlorométhyle, CN et le groupe CO-O-alkyle à 2 à 5 atomes de carbone, alcoxyalkyle ou alkylthioalkyle ayant chacun 2 à 4 atomes de carbone et $R^2$ halogénalcényle, alcényle, hydrogène ou un reste alkyle à 1 à 4 atomes de carbone, par chauffage d'esters d'acide 3,5-dichlorophénylcarbamoyloxyacétique de formule

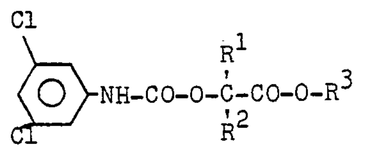

dans laquelle $R^1$ et $R^2$ ont les significations sus-indiquées et $R^3$ représente un reste alkyle à 1 à 10 atomes de carbone ou un reste cyclohexyle, en présence d'un catalyseur basique, à des températures comprises entre 20 et 200 °C et séparation d'alcool de formule

$R^3$—OH

où $R^3$ a la signification sus-indiquée, caractérisé par le fait qu'on effectue le chauffage en présence d'un alcool de formule

$R^4$—OH

où $R^4$ représente un reste alkyle à 1 à 10 atomes de carbone, hydroxyéthyle ou un reste cyclohexyle et est identique à ou différent de $R^3$, et qu'on sépare du mélange de réaction, à des températures comprises entre − 30 et + 90 °C, selon un procédé connu, la oxazolidine-2,4-dione qui a précipité, sous forme solide, du mélange de réaction.

2. Procédé selon la revendication 1, caractérisé par le fait qu'on effectue la réaction avec des composés dans lesquels $R^1$ représente vinyle ou méthoxyméthyle, $R^2$ méthyle et $R^3$ alkyle à 1 à 4 atomes de carbone.

3. Procédé selon la revendication 1, caractérisé par le fait que $R^4$ représente alkyle à 1 à 4 atomes de carbone.

4. Procédé selon la revendication 1, caractérisé par le fait qu'on utilise, comme catalyseur, une amine tertiaire.